# EUROPÄISCHE PATENTSCHRIFT

(11) **EP 3 782 682 B1**
(45) Veröffentlichungstag und Bekanntmachung des Hinweises auf die Patenterteilung: **27.09.2023**
(21) Anmeldenummer: 19192926.4
(22) Anmeldetag: 21.08.2019
(51) Int. Cl.: A61M 15/00, G16H 20/10

(54) **INHALATOR UND AUSWERTUNGSEINHEIT HIERFÜR**
INHALER AND EVALUATION UNIT FOR SAME
INHALATEUR ET UNITÉ D'ÉVALUATION CORRESPONDANT

(43) Veröffentlichungstag der Anmeldung: 24.02.2021
(73) Patentinhaber: Aptar Radolfzell GmbH, 78315 Radolfzell (DE)
(72) Erfinder: Helmlinger, Michael, 78315 Radolfzell-Böhringen (DE); Karge, Marius, 78333 Stockach (DE); Schmid, Kevin, 78462 Konstanz (DE)
(74) Vertreter: Witte, Weller und Partner Patentanwälte mbB Stuttgart

(56) Entgegenhaltungen:
- EP-A1- 2 221 079
- WO-A1-2017/205824
- US-A1- 2016 144 141

## Beschreibung

### ANWENDUNGSGEBIET UND STAND DER TECHNIK

Die Erfindung betrifft einen Inhalator zur Abgabe eines inhalationsfähigen Mediums, insbesondere einer zerstäubten Flüssigkeit. Ein erfindungsgemäßer Inhalator zeichnet sich durch eine Auswertungseinheit aus, die an einem Container des Inhalators angebracht ist.

Ein Inhalator, der erfindungsgemäß mit einer Auswertungseinheit entsprechend der Erfindung versehen wird, kann insbesondere als MDI-Spender (Metered Dose Inhaler) ausgebildet sein. Solche MDI-Spender weisen üblicherweise eine in etwa L-förmige Formgebung auf und verfügen über einen vertikal ausgerichteten Hauptkörper, der einen Aufnahmeraum für eine Containereinheit beinhaltet, und ein sich hieran am unteren Ende anschließendes Mundstück, das gegenüber dem Hauptkörper angewinkelt ist.

Die Nutzung von gattungsbildenden Inhalatoren erfolgt derart, dass ein Benutzer das Mundstück zwischen die Lippen nimmt und hierdurch einatmet, so dass Luft durch das Gehäuse des Inhalators eingesogen wird. Diese Luft strömt üblicherweise durch einen Spalt zwischen der Containereinheit und einer umgebenden Wandung des Aufnahmeraums in das Gehäuse ein. Der Strömungswiderstand ist dabei gering, so dass es keiner nennenswerten Anstrengung beim Einatmen bedarf. Während des Einatmens drückt der Benutzer von oben auf die Containereinheit und bewirkt dadurch einen Austrag von zerstäubter Flüssigkeit in den Luftstrom, so dass die inhalierte Luft mit Tröpfchen der Flüssigkeit vermengt und vom Benutzer inhaliert wird.

Die gewünschte medizinische Wirkung der Inhalation hängt in erheblichem Maße davon ab, dass der Benutzer die bestimmungsgemäßen Parameter einhält, also insbesondere stark genug einatmet und den Austrag der Flüssigkeit zum richtigen Zeitpunkt bewirkt.

Aus dem Stand der Technik sind bereits Spender bekannt, die die Einhaltung dieser Parameter überwachen und ggf. Rückmeldung an den Benutzer geben bzw. ggf. auch Daten zur Nutzung des Spenders und zur Einhaltung der Paramater versenden, insbesondere zur Auswertung durch einen Arzt.

Hierbei sind auch bereits Auswertungseinheiten bekannt, die in der auch bei der Erfindung vorgesehenen Art und Weise auf einen Containerboden aufgesetzt werden, um gemeinsam mit dem Container vom Benutzer gegenüber dem Gehäuse des Inhalators niedergedrückt zu werden.

Aus der WO 2017/205824 A1 ist eine Auswertungseinheit für einen Inhalationsspender bekannt, der über ein Druckrohr verfügt, welches aus dem Ringspalt eines Inhalators zwischen Gehäusewandung und Container zu einer Platine führt, an der ein Drucksensor vorgesehen ist. Es dient der Erfassung eines Drucks im genannten Ringspalt.

Aus der US 2016/0144141 A1 ist eine Auswertungseinheit für einen Inhalationsspender bekannt, bei dem die zu inhalierende Luft durch Belüftungsschlitze der Auswertungseinheit eingesogen wird und bei der weiterhin ein interner Drucksensor vorgesehen ist, der zur Erfassung des Unterdrucks ausgebildet ist, der sich beim Ansaugen von Luft durch den Benutzer in der Auswertungseinheit einstellt. WO2015109259 beschreibt einen Inhalator mit einem Ringspalt und einem Sensor wo entlang Luft angesaugt wird.

Problematisch an der Erfassung des Einatmens durch einen Benutzer ist es, dass es schwierig ist, den Luftstrom vollständig zu erfassen. Insbesondere besteht die Gefahr, dass eine Messeinrichtung zur Erfassung des Luftstroms den Strömungswiderstand deutlich erhöht und somit für das korrekte Einatmen durch den Benutzer eher nachteilig ist.

### AUFGABE UND LÖSUNG

Aufgabe der Erfindung ist es, eine Auswertungseinheit sowie einen Inhalator mitsamt einer Auswertungseinheit zur Verfügung zu stellen, die ein angenehmes und müheloses Einatmen gestatten, gleichzeitig jedoch ein Erfassen der eingeatmeten Luftmenge mit ausreichender Sicherheit gestatten.

Erfindungsgemäß wird hierfür zum einen ein Inhalator vorgeschlagen, der übereinstimmend mit bekannten Inhalatoren über ein Gehäuse mit einem Mundstück und einem von einer Aufnahmeraumwandung umgebenen Aufnahmeraum für eine Containereinheit verfügt. Der Aufnahmeraum ist an einem Ende offen ausgebildet, so dass die Containereinheit an diesem offenen Ende in den Aufnahmeraum einsetzbar ist.

Die Containereinheit selbst verfügt in gattungstypischer Weise über einen Auslassstutzen, der kommunizierend an einen Austragkanal des Gehäuses angeschlossen ist, sowie über einen Container zur Lagerung des Mediums vor dem Austrag. Durch Verlagerung des Containers gegenüber dem Auslassstutzen kann ein Auslassventil der Containereinheit geöffnet werden. Insbesondere kann der Container unter Druck stehen, um beim Öffnen des Auslassventils automatisch das enthaltene Medium abzugeben, insbesondere eine zuvor isolierte definierte Dosis des Mediums. Es sind auch Gestaltungen mit Pumpen mit zwei Ventilen und dazwischenliegender Pumpkammer bekannt.

Der Container weist nach dem Einsetzen zum offenen Ende des Aufnahmeraums, so dass er von einem Benutzer niederdrückbar ist, um hierdurch das Auslassventil zu öffnen und Medium durch den Austragkanal bis zu einer Austragöffnung am Ende des Austragskanal austreten zu lassen. Der Container ragt im unbetätigten Zustand vorzugweise aus dem Aufnahmeraum hinaus.

Der Austrag erfolgt bestimmungsgemäß bei gleichzeitiger Inhalation durch den Benutzer. Es ist daher zwischen dem Container und der Aufnahmeraumwandung ein Ringspalt vorgesehen, durch den hindurch Luft während der Inhalation angesogen wird. Diese Luft vermengt sich bestimmungsgemäß mit durch die Austragöffnung ausströmendem Medium aus dem Container. Das Gemisch wird vom Benutzer inhaliert.

Wie eingangs bereits genannt, verfügt der erfindungsgemäße Inhalator über eine Auswertungseinheit zur Auswertung der Nutzung. Diese Auswertungseinheit ist auf dem vom Auslassstutzen weg weisenden Ende des Containers aufgesetzt, welches zum offenen Ende des Aufnahmeraums weist. Die Auswertungseinheit ist gemeinsam mit dem Container niederdrückbar. Die Auswertungseinheit ist erfindungsgemäß zur Erfassung der am Mundstück und insbesondere durch den Ringspalt eingesogenen Luft ausgebildet und umfasst hierfür einen Messkanal mit einem Messkanaleingang und einem Messkanalausgang, der einen Teil eines ersten Strömungspfades für aus einer Umgebung in das offene Ende des Aufnahmeraums einströmende Luft bildet und der zwischen dem Messkanaleingang und dem Messkanalausgang über einen Durchflusssensor zur Erfassung der durch den Messkanal strömenden Luft verfügt. Neben dem ersten Strömungspfad durch den Messkanal existiert weiterhin ein zweiter Strömungspfad für aus einer Umgebung in das offene Ende des Aufnahmeraums einströmende Luft, wobei dieser zweiten Strömungspfad nicht durch den Messkanal führt. Er führt stattdessen am Messkanal vorbei durch den Ringspalt und/oder durch anderweitige Öffnungen des Gehäuses des Inhalators.

Der erste Strömungspfad und der zweite Strömungspfad sind derart aufeinander abgestimmt, dass der Strömungswiderstand des ersten Strömungspfades mindestens dem Strömungswiderstand des zweiten Strömungspfades entspricht. Dies bedeutet, dass beim Ansaugen von Luft am Mundstück maximal die Hälfte der Luft durch den Messkanal geleitet wird.

Ein erfindungsgemäßer Inhalator mit einer erfindungsgemäßen Auswertungseinheit zeichnet sich dadurch aus, dass die bei der Inhalation aus einer Umgebung in das Gehäuse einströmende und zum Mundstück gelangende Luft zumindest zur Hälfte und vorzugsweise zum weit überwiegenden Teil entlang des genannten zweiten Strömungspfades eingesogen wird, der nicht durch den Messkanal führt. Soweit im Kontext der Erfindung vom zweiten Strömungspfad gesprochen wird, meint dies die Gesamtheit der Strömungspfade, die aus einer Umgebung unter Umgehung des Messkanals zum Mundstück führen.

Nur maximal die Hälfte der Luft, vorzugweise ein deutlich geringerer Anteil, wird durch den Messkanal eingesogen und unterliegt somit dem dortigen vergleichsweise hohen Strömungswiderstand.

Der Erfindung liegt die Erkenntnis zugrunde, dass es in ausreichender Genauigkeit möglich ist, die gesamte eingesogene Luftmenge, also die entlang des ersten Strömungspfades durch den Messkanal und entlang des zweiten Strömungspfades am Messkanal vorbei eingesogene Luftmenge zu ermitteln, indem alleine der Luftstrom entlang des ersten Strömungspfades ausgewertet wird. Im einfachsten Falle besteht näherungsweise ein linearer Zusammenhang zwischen den Luftströmen. Ist dies nicht der Fall, so kann mittels darüber hinausgehender formelmäßiger Zusammenhänge oder über in Tabellen abgelegte Testreihen ausgehend vom Luftstrom durch den ersten Strömungspfad der Luftstrom im zweiten Strömungspfad errechnet werden. Ja nach Art der Auswertung ist es nicht erforderlich, den Luftstrom durch den zweiten Strömungspfad zu errechnen, da die Auswertung alleine des erfassten Luftstroms des ersten Strömungspfades für die Bewertung der Inhalation ausreicht.

Die Auswertungseinheit, die den Messkanal zur Verfügung stellt, ist derart gestaltet, dass sie endseitig auf den Container der Containereinheit aufgesetzt ist. Statt zur Abgabe auf den Containerboden zu drücken, drückt der Benutzer auf dieser Auswertungseinheit, die dann gemeinsam mit dem Container niedergedrückt wird. Die Auswertungseinheit, die auch elektronische Komponenten umfasst und somit vergleichsweise teuer ist, ist üblicherweise zur wiederholten Verwendung mit mehreren Inhalatoren oder zumindest mehreren Containereinheiten vorgesehen, so dass nach einem Austausch des Inhalators bzw. der Containereinheit die von der vorherigen Containereinheit getrennte Auswertungseinheit auf die neue Containereinheit aufgesetzt wird.

Der Messkanal ist in diese Auswertungseinheit integriert und erstreckt sich vom genannten Messkanaleingang bis zum genannten Messkanalausgang. Der Messkanaleingang und der Messkanalausgang sind voneinander beabstandet. Der Messkanal selbst ist umfänglich geschlossen und beinhaltet den Durchflusssensor, der auf verschiedene Arten ausgebildet sein kann, wobei ein thermischer Durchflusssensor bevorzugt ist. Die mittlere Querschnittsfläche des Messkanals beträgt vorzugsweise zwischen 0,2 mm² und 4 mm². Die engste Querschnittsfläche des Messkanals beträgt vorzugsweise zwischen 0,1 mm² und 2 mm². Die Länge beträgt vorzugsweise zwischen 10 mm und 60 mm.

Wie bereits beschrieben, entspricht der Strömungswiderstand durch den zweiten Strömungspfad am Messkanal vorbei maximal jenem des ersten Strömungspfades. Vorzugsweise ist der Strömungswiderstand durch den ersten Strömungspfad jedoch erheblich höher als der des zweiten Strömungspfades und entspricht vorzugsweise mindestens dem zehnfachen des Strömungswiderstandes durch den zweiten Strömungspfad, insbesondere vorzugsweise mindestens dem 25-fachen und weiterhin bevorzugt mindestens dem 35-fachen des Strömungswiderstandes durch den zweiten Strömungspfad.

Es wird bevorzugt, dass der Strömungswiderstand entlang des ersten Strömungspfades mindestens 1.000.000 N · s / m⁵ beträgt und der Strömungswiderstand entlang des zweiten Strömungspfades maximal 1.000.000 N · s / m⁵ beträgt. Vorzugsweise liegt der Strömungswiderstand entlang des ersten Strömungspfades bei mindestens 5.000.000 N · s / m⁵. Weiterhin vorzugsweise liegt der Strömungswiderstand entlang des zweiten Strömungspfades bei maximal 500.000 N · s / m⁵.

Trotz der Tatsache, dass dieser vorzugsweise gegebene große Unterschied des Strömungswiderstand dazu führt, dass nur ein geringer Anteil der eingesogenen Luft entlang des ersten Strömungspfades und durch den Messkanal strömt, hat sich gezeigt, dass bei ausreichend genauer Erfassung des Luftstroms durch den ersten Strömungspfad die eingesogene Gesamtluftmenge sicher abschätzbar ist.

Um die Gesamtluftmenge bzw. den Gesamtluftstrom mit der gewünschten Genauigkeit errechnen zu können bzw. um alleine auf Basis des gemessenen Luftstroms eine zweckmäßige Auswertung zu gestatten, ist es von Vorteil, wenn der Luftstrom, der den Messkanal durchströmt, vergleichsweise genau erfasst wird. Für die Realisierung des Durchflusssensors sind grundsätzlich sehr verschiedene Sensoren verwendbar. So kommt beispielsweise in Frage, dass im Messkanal ein gegen eine Rückstellkraft auslenkbares Element vorgesehen ist, dessen Auslenkung erfasst und als Maß für den Luftstrom herangezogen wird. Auch kann beispielsweise vorgesehen sein, dass im Messkanal ein rotorähnliches Element drehbar vorgesehen ist, dessen Rotationsgeschwindigkeit erfasst und als Maß für den Luftstrom herangezogen wird.

Es hat sich jedoch gezeigt, dass der vorzugsweise nur geringe Anteil von Luft, der durch den Messkanal geführt ist, besonders genau und damit besonders vorteilhaft mittels eines Durchflusssensor ermittelt werden kann, der als thermischer / kalorimetrischer Durchflusssensor ausgebildet ist. Ein solcher kalorimetrischer Sensor verfügt über zwei Temperatursensoren und ein dazwischenliegendes Heizelement.

Der thermische Durchflusssensor ist dafür ausgebildet, mittels des Heizelements eine erwärmte Zone im Sensorkanal zu erzeugen und an den beiden Temperatursensoren den dadurch bewirkten Temperaturanstieg zu ermitteln. Fließt keine Luft durch den Sensorkanal, so wird aufgrund des identischen Abstandes zum Heizelement an beiden Temperatursensoren die gleiche Temperatur erfasst. Fließt jedoch Luft durch den Sensorkanal, so ist die Erwärmung asymmetrisch. Je größer der Temperaturunterschied ist, desto mehr Luft strömt durch den Sensorkanal.

Der Messkanal ist vorzugsweise zumindest teilweise in einem Messkanalstutzen vorgesehen, der in Längsrichtung vom Messkanal durchdrungen ist. Dieser Messkanalstutzen erstreckt sich von der Auswertungseinheit in Richtung des Aufnahmeraums des Inhalators. Er weist vorzugsweise eine sich in Richtung einer Auslassöffnung des Messkanals verjüngende Formgebung auf, insbesondere eine sich stetig verjüngende Formgebung. Dies erleichtert das Einführen des Messkanalstutzens in den Ringspalt zwischen Container und Aufnahmeraumwandung. Das Messkanalende ist vorzugsweise am distalen Ende dieses Messkanalstutzens vorgesehen.

Die Auswertungseinheit kann bezüglich ihrer Außenkontur im Wesentlichen rotationssymmetrisch ausgebildet sein. Von Vorteil ist allerdings eine Gestaltung, bei der die Auswertungseinheit einen Kopfabschnitt aufweist, der oberhalb des Containers angeordnet ist und auf dessen Boden aufliegt, wobei zusätzlich ein sich vom Kopfbereich in einem Winkelbereich < 360° in Richtung der Aufnahmeraumwandung erstreckenden Schürzenabschnitt vorgesehen ist, der sich seitlich des Containers nach unten erstreckt. Dieser Schürzenabschnitt ist dabei vorzugsweise außenseitig des vom Messkanal durchdrungenen Messkanalstutzens angeordnet.

Der Schürzenabschnitt kann eine Mehrzahl von Funktionen erfüllen. Hierzu gehört, dass er durch die Anordnung außenseitig des Messkanalstutzens diesen schützen kann, insbesondere in einer Situation, in der die Auswertungseinheit zum Zwecke des Tausches der Containereinheit von der Containereinheit getrennt ist.

Insbesondere ist der genannte Aufbau mit dem Schürzenabschnitt gut zur Erzielung einer Verdrehsicherung geeignet. Es ist bevorzugt, dass die Auswertungseinheit und die Aufnahmeraumwandung des Aufnahmeraums bezüglich ihrer Formgebung derart aufeinander abgestimmt sind, dass die Auswertungseinheit nur in einer definierten Drehstellung oder einem definierten Drehstellungsbereich auf den Container aufsetzbar ist. Es hat sich gezeigt, dass für eine ausreichend genaue Berechnung des Gesamtluftstroms auf Basis des erfassten Luftstroms entlang des ersten Strömungspfades bzw. für die Bewertung der Inhalation alleine auf Basis des Luftstroms entlang des ersten Strömungspfades erheblich ist, dass der erste Strömungspfad bzgl. seines Verlaufes weitgehend unveränderlich ist. Durch die genannte Verdrehsicherung wird erreicht, dass der Messkanal, insbesondere der Messkanaleingang und der Messkanalausgang, stets relativ zu den anderen Komponenten des Inhalators an gleichbleibender Stelle sind. So kann insbesondere vorgesehen sein, dass der Messkanaleingang und der Messkanalausgang auf der dem Mundstück abgewandten Seite des Inhalators vorgesehen sind, da hier die Beeinflussung des Messergebnisses durch am Mundstück auftretende Turbulenzen gering ist.

Insbesondere zur Erzielung dieser Verdrehsicherung ist es bevorzugt, wenn die Auswertungseinheit den bereits genannten Kopfabschnitt sowie den sich hiervon in Richtung der Aufnahmeraumwandung erstreckenden Schürzenabschnitt aufweist. Der Schürzenabschnitt kann dabei eine nach innen weisende Formgebung aufweisen, die an eine nicht rotationssymmetrische Außenform der Aufnahmeraumwandung drehsichernd formschlüssig angepasst ist. Vorzugweise überlappt der Schürzenabschnitt die Aufnahmeraumwandung bereits im nicht niedergedrückten Zustand.

Zusätzlich oder alternativ dazu, dass die Verdrehsicherung mittels eines äußeren Übergreifens der Aufnahmeraumwandung durch den Schürzenabschnitt erzielt wird, kann auch vorgesehen sein, dass der Ringspalt zwischen dem Container und einer Innenseite der Aufnahmeraumwandung eine nichteinheitliche lichte Weite aufweist und nicht umlaufend ausreichend für die Aufnahme des vom Messkanal durchdrungenen Messkanalstutzens dimensioniert ist. Der Ringspalt ist demnach bei einer solchen Ausgestaltung nur in einem bestimmten Winkelbereich ausreichend groß, so dass nur hier beim Aufsetzen der Auswertungseinheit der Messkanalstutzen einführbar ist.

Insbesondere von Vorteil ist es, wenn der Messkanal und/oder der Schürzenabschnitt bei auf den Container aufgesetzter Auswertungseinheit auf gegenüberliegender Seite zum Mundstück angeordnet ist. Dies führt zu einer angenehmeren Handhabung des Inhalators, da der Schürzenabschnitt während der Benutzung des Inhalators nicht in Richtung des Gesichts des Nutzers weist. Weiterhin ist diese Anordnung in der oben erwähnten Weise von Vorteil, um für die Messung nachteilige Turbulenzen am Messkanal zu vermeiden.

Bevorzugt ist weiterhin eine Gestaltung, bei der der Messkanal und insbesondere der vom Messkanal durchdrungene Messkanalstutzen zumindest bei niedergedrücktem Container im Ringspalt zwischen Aufnahmeraumwandung und Container angeordnet sind. Hierdurch ist gewährleistet, dass ein zumindest im Wesentlichen reproduzierbarer Anteil der inhalierten Luft durch den Messkanal strömt. Insbesondere vorzugsweise ist die Auslassöffnung des Messkanals bereits bei nicht niedergedrücktem Container im Ringspalt angeordnet, so dass die Gefahr verringert ist, dass der Messkanal oder der Messkanalstutzen beim Niederdrücken des Containers mit der Aufnahmeraumwandung kollidiert.

Insbesondere bevorzugt ist es, wenn die Auswertungseinheit eine Durchflusssensoreinheit aufweist, die über ein Sensorgehäuse mit nichtlinearem Sensorkanal verfügt. Hierunter ist ein Sensorkanal zu verstehen, der mindestens einmal umgelenkt wird. Eine solche Durchflusssensoreinheit kann bei der Montage der Auswertungseinheit recht einfach gehandhabt werden und gestattet es insbesondere, auf einer Unterseite einer horizontalen Hauptplatine im Kopfabschnitt der Auswertungseinheit angebracht zu werden, wobei ein Teil des Sensorkanals parallel zur Hauptplatine verläuft, während ein Ende oder vorzugsweise beide Enden des Sensorkanals abknicken und insbesondere nach unten in Richtung des Aufnahmeraums und des dortigen Ringspaltes weisen.

Die Ausrichtung der Enden der Durchflusssensoreinheit und insbesondere des stromabwärtigen Endes des Sensorkanals gestattet es, dass während der Montage an diesen unmittelbar anschließend ein geradlinig erstreckter Messkanalabschnitt angesteckt wird, der durch den genannten Messkanalstutzen gebildet wird. Der Messkanalstutzen kann daher als recht einfaches und einfach entformbares Kunststoffteil ausgebildet sein. Insbesondere kann der Messkanalstutzen einstückiger Teil eines Innenbauteils der Auswertungseinheit sein, das gemeinsam mit einem Außenbauteil einen Innenraum zur geschützten Anordnung elektronischer Komponenten bildet.

Die Luft, die entlang des ersten Strömungspfades einströmt und somit den Messkanal durchquert und hier erfasst wird, kann durch einen Spalt zwischen Auswertungseinheit und Aufnahmeraumwandung einströmen. Alternativ oder zusätzlich ist es jedoch auch möglich, das Gehäuse der Auswertungseinheit mit mindestens einer Durchbrechung zu versehen, durch die Luft aus einer Umgebung zu einem Messkanaleinlass strömen kann. Solche Durchbrechungen können von Vorteil sein, da durch sie die Luft einströmen kann, ohne hierbei das Ausströmen von Luft aus dem Messkanal zu beeinflussen. Die Messergebnisse der Durchflusssensoreinheit sind daher in besonders hohem Maße reproduzierbar.

Die mindestens eine Durchbrechung zum Einströmen der Luft kann an einer Oberseite eines Kopfabschnitts der Auswertungseinheit vorgesehen sein. Alternativ oder zusätzlich kann mindestens eine Durchbrechungzum Einströmen der Luft an einer Mantelfläche der Auswertungseinheit, insbesondere an dem sich in Richtung der Aufnahmeraumwandung erstreckenden Schürzenabschnitt, vorgesehen sein. Einströmöffnungen, die die Auswertungseinheit und insbesondere ein äußeres Gehäusebauteil der Auswertungseinheit durchbrechen, können vorteilhaft sein, da sie aufgrund weniger erforderlicher Umlenkungen des Luftstroms mit einer geringeren Gefahr von turbulenten Strömungen einhergehen. Dies ist in Hinblick auf die Messgenauigkeit von Vorteil.

Der zweite Strömungspfad, der erfindungsgemäß einen Strömungswiderstand aufweist, der maximal dem des ersten Strömungspfades entspricht, vorzugsweise jedoch viel geringer ist, verläuft vorzugsweise ebenfalls zumindest abschnittsweise durch einen Ringspalt zwischen dem Container und der Aufnahmeraumwandung, wenngleich auch anderweitige Öffnungen im Gehäuse des Inhalators das Einströmen von Luft am Messkanal vorbei gestatten können. Der Einlass in diesen zweiten Strömungspfad wird vorzugsweise durch einen Einströmspalt gebildet, der auf der einen Seite durch ein oberes Ende der Aufnahmeraumwandung und auf der anderen Seite durch einen Kopfabschnitt der Auswertungseinheit gebildet wird.

Dieser Einströmspalt weist vorzugsweise eine lichte Breite von mindestens 1 mm auf, wobei dies insbesondere vorzugsweise sowohl im ungedrückten Zustand des Containers als auch im gedrückten Zustand gilt. Der Einströmspalt kann einheitlich vollständig umlaufend vorgesehen sein. Von Vorteil ist jedoch, wenn er vom bereits erwähnten Schürzenabschnitt der Auswertungseinheit unterbrochen ist, wobei dies nicht bedeuten muss, dass keinerlei Luft unterhalb des Schürzenabschnitts einströmen kann und entlang des zweiten Strömungspfades zum Mundstück gelangen kann. Jedoch ist der lichte Spalt unter dem Schürzenabschnitt vorzugsweise kleiner.

Der vorzugsweise vom Schürzenabschnitt zumindest teilweise beschränkte Einströmspalt umgibt den Container vorzugsweise mindestens in einem Winkelbereich von 180°, vorzugweise in einem Winkelbereich zwischen 200° und 340°. Dies bedeutet, dass in diesem Winkelbereich die Auswertungseinheit das Einströmen von Luft in den Ringspalt gegenüber der Situation ohne aufgesetzte Auswertungseinheit nicht wesentlich beschränkt und insbesondere der Strömungswiderstand in diesem Winkelbereich durch die Anwesenheit der Auswertungseinheit maximal um 10% erhöht wird.

Die Erfindung betrifft neben dem Inhalator als Ganzem auch eine Auswertungseinheit für einen Inhalator zur Abgabe eines inhalationsfähigen Mediums. Alle oben stehenden Informationen in Hinblick auf diese Auswertungseinheit gelten nicht nur für die Auswertungseinheit als Teil eines Inhalators, sondern auch für die erfindungsgemäße Auswertungseinheit als solche, die zur Ankopplung an einen gattungstypischen Inhalator vorgesehen ist. Ebenso sind die im Folgenden beschriebenen Merkmale der Auswertungseinheit vorzugsweise auch bei der oben beschriebenen Auswertungseinheit als Teil eines Inhalators vorgesehen.

Eine erfindungsgemäße Auswertungseinheit ist zur Verwendung mit einem Inhalator vorgesehen, der in der insbesondere für MDI-Spender üblichen Art über ein Gehäuse mit einem Mundstück und einem von einer Aufnahmeraumwandung umgebenen Aufnahmeraum mit darin eingesetzter Containereinheit verfügt. Wie oben bereits beschrieben, ist dabei zwischen einem Container der Containereinheit und der Aufnahmeraumwandung des Aufnahmeraums ein Ringspalt vorgesehen, durch den hindurch Luft während der Inhalation angesogen wird, die sich mit dem durch eine Austragöffnung ausströmenden Medium aus dem Container vermengt.

Die Auswertungseinheit verfügt über einen Befestigungsabschnitt mit einem Einschubbereich zum Einschub des Bodens des Containers in eine Einschubrichtung. Dieser Befestigungsabschnitt ist vorzugsweise dafür ausgebildet, mit dem Container eine Klemmverbindung einzugehen. Vorzugsweise ist der Einschubbereich zu diesem Zweck auf die Außenkontur und insbesondere den Durchmesser des Containers angepasst. Alternativ dazu kann der Befestigungsabschnitt auch verstellbar sein, um auf Container unterschiedlicher Maße aufgesetzt zu werden. Hierzu kann die Auswertungseinheit einen mindestens zweiteiligen Befestigungsabschnitt aufweisen, der einen Einschubbereich begrenzt, wobei durch Verlagerung der zwei Teile die Größe des Einschubbereichs anpassbar ist.

Die erfindungsgemäße Auswertungseinheit verfügt weiterhin über einen sich parallel zur Einschubrichtung erstreckenden Messkanalstutzen, der von dem Messkanal durchdrungen ist, durch den ein Anteil der während der Inhalation einströmenden Luft in den Ringspalt gelangen kann, wobei der Messkanalstutzen dafür vorgesehen ist, von oben in den Ringspalt hineinzuragen.

Im Messkanal ist ein Durchflusssensor zur Erfassung der durch den Messkanal strömenden Luft vorgesehen. Wie oben bereits beschrieben, kommen hier verschiedene Arten von Durchflusssensoren in Frage. Bevorzugt ist die Verwendung des bereits beschriebenen thermischen Durchflusssensors, der ein Heizelement aufweist und den hierdurch bewirkten Temperaturanstieg an zwei Temperatursensoren stromabwärts und stromaufwärts des Heizelementes erfasst. Wie oben erwähnt beträgt die mittlere Querschnittsfläche des Messkanals vorzugsweise zwischen 0,2 mm² und 4 mm² und die Länge des Messkanals vorzugsweise zwischen 10 mm und 60 mm.

Wie eingangs bereits beschrieben, strömt nur ein Anteil, vorzugsweise nur ein geringer Anteil, der vom Nutzer inhalierten Luft durch den Messkanal. Nur dieser Anteil wird vom Durchflusssensor erfasst.

Die Auswertungseinheit weist vorzugsweise eine Steuerschaltung auf, die Ausgangssignale des Durchflusssensors erhält und zur Auswertung dieser Strömungssignale vorgesehen ist. Dabei ist die Auswertungseinheit dafür ausgebildet, anhand des Luftstroms entlang des ersten Strömungspfades eine Berechnung des Gesamtluftstroms entlang des ersten und des zweiten Strömungspfades vorzunehmen. Im einfachsten Falle wird der Gesamtluftstrom dadurch berechnet, dass der erfasste Luftstrom mit einem anhand der Strömungswiderstände ermittelten Faktor multipliziert wird.

Vorzugsweise ist die Auswertungseinheit auch dafür ausgebildet, den Gesamtluftstrom anhand eines nichtlinearen Verhältnisses zwischen dem Luftstrom im ersten Strömungspfad und dem Gesamtluftstrom zu berechnen. Es hat sich gezeigt, dass je nach Geometrie des Messkanals eine Berechnung des Gesamtluftstroms mittels eines einfachen Faktors nicht ausreicht. Die Steuerschaltung der Auswertungseinheit kann daher vorzugsweise anhand darin hinterlegter Tabellendaten oder nichtlinearer Formelzusammenhänge den Gesamtluftstrom ermitteln.

Die Auswertungseinheit ist vorzugsweise dafür ausgebildet, den aufsummierten Luftstrom entlang beider Strömungspfade oder den erfassten Luftstrom entlang des ersten Strömungspfades in den Kontext weiterer erfasster Daten zu setzen. Insbesondere kann die Auswertungseinheit den Luftstrom in Hinblick auf seine zeitliche Abstimmung mit dem Niederdrücken des Containers auswerten. Zu diesem Zwecke weist die Auswertungseinheit vorzugsweise auch mindestens einen Sensor auf, der die Kraftbeaufschlagung der Auswertungseinheit und/oder das Niederdrücken der Auswertungseinheit und des Containers erfassen kann. Dieser Sensor kann insbesondere ein Taster oder eine als Kraft- oder Drucksensor wirkende Folie sein, der bzw. die zwischen eine Oberseite der Auswertungseinheit und dem Containerboden angeordnet ist, so dass beim Niederdrücken von Auswertungseinheit und Container die hier wirkende Kraft erfassbar ist und Rückschlüsse auf den Austrag gestattet. Alternativ kann der Sensor auch in Art eines Tasters oder dergleichen vorgesehen sein, der die Relativverlagerung der Auswertungseinheit gegenüber der Wandung des Aufnahmeraums erfasst, indem der Taster an einer Gegenfläche der Wandung eingedrückt wird und hierdurch den Austrag erfassbar macht.

Die Auswertungseinheit kann auswerten, ob zum Zeitpunkt des Austrags ein ausreichender Luftstrom vom Nutzer inhaliert worden ist und ob die Inhalation ausreichend lange nach dem Austrag fortgesetzt wurde, so dass das Medikament, insbesondere die zerstäubte Flüssigkeit, ihr bestimmungsgemäßes Ziel, insbesondere die Lunge, erreicht hat.

Die durch die Auswertungseinheit erfassten Sensordaten und Ergebnisdaten von auf Basis der Sensordaten erfolgten Auswertungen können alleine dafür verwendet werden, zur späteren Analyse gespeichert zu werden oder an einen Zentralserver übermittelt zu werden, um dritten Personen wie dem Arzt hierauf Zugriff zu ermöglichen. Vorzugsweise ist die Auswertungseinheit jedoch dafür ausgebildet, einem Benutzer eine optische, haptische oder akustische Rückmeldung zu den erfassten oder ermittelten Daten zu geben, insbesondere durch ein Display, Status-LEDs oder einen Lautsprecher. Diese Rückmeldung kann je nach Ausgestaltung bereits während des Inhalationsvorgangs erfolgen, so dass der Nutzer die Nutzung unmittelbar daran orientiert anpassen kann. Die Rückmeldung kann jedoch auch nach dem Inhalationsvorgang erfolgen, so dass der Nutzer mitgeteilt bekommt, welche Schlüsse aus den erfassten Daten der abgeschlossenen Inhalation gezogen werden können.

Die Auswertungseinheit verfügt vorzugsweise über ein einstückiges Innenbauteil, welches einen Einschubbereich zur Befestigung am Container unmittelbar begrenzt und welches zumindest abschnittsweise den Messkanal definiert, insbesondere vom Messkanal durchdrungen ist. Der Messkanalabschnitt, der durch das genannte Innenbauteil gebildet ist, verläuft vorzugsweise rein linear, so dass das Innenbauteil als einfach entformbares Kunststoffbauteil ausgebildet sein kann. Insbesondere vorzugsweise bildet das Innenbauteil einstückig den bereits erwähnten Messkanalfortsatz, der sich bis in den den Container umgebenden Ringspalt erstreckt.

Vorzugsweise ist ein Außengehäuse der Auswerteeinheit durch das genannte Innenbauteil sowie ein korrespondierendes Außenbauteil gebildet, die gemeinsam einen Innenbereich definieren, in welchem verschiedene elektronische Komponenten und/oder Sensoren angeordnet sein können. Insbesondere kann sich auch der Messkanal durch diesen Innenbereich hindurch erstrecken. Das Innenbauteil und das Außenbauteil können beispielsweise durch eine Verrastung miteinander verbunden sein. Insbesondere vorzugsweise kann die Auswertungseinheit eine Aufnahme für eine Batterie aufweisen, insbesondere eine Knopfzelle, wobei vorzugsweise ein ausziehbarer Batterieträger zur Aufnahme der Batterie vorgesehen ist. Insbesondere kann der Batterieträger einen Riegelabschnitt bilden, mittels dessen das Innenbauteil und das Außenbauteil der Auswertungseinheit miteinander verriegelbar sind.

Neben dem erfindungswesentlichen Durchflusssensor sowie dem bereits erwähnten Sensor zur Erfassung einer von oben auf die Auswertungseinheit ausgeübten Betätigungskraft relativ zum Container kann eine erfindungsgemäße Auswertungseinheit noch weitere Sensoren aufweisen. Hierzu gehört insbesondere ein Schüttelsensor, üblicherweise realisiert durch einen Beschleunigungssensor, der erfassen kann, ob der Benutzer vor Verwendung des Spenders den Container ausreichend geschüttelt hat. Dieser Sensor kann je nach Ausgestaltung auch anderweitige Beschleunigungen erfassen, insbesondere auch solche, die mit dem Ergreifen des Spenders durch einen Benutzer einhergehen.

Die neben dem Durchflusssensor anderweitigen Sensoren der Auswertungseinheit können insbesondere auch derart Verwendung finden, dass der vergleichsweise viel Energie benötigende Durchflusssensor erst in Abhängigkeit der Ausgangsdaten dieser anderweitigen Sensoren aktiviert wird.

Zu den elektronischen Komponenten der Auswertungseinheit gehört vorzugsweise auch eine Kommunikationseinrichtung zur Kommunikation mit einer externen Kommunikationsstelle, wobei es sich insbesondere vorzugsweise um eine drahtlos arbeitende Kommunikationseinrichtung handelt. Hierbei kommen Kommunikationseinrichtungen der üblichen Standards in Frage, also insbesondere nach einem WiFi-Standard (IEEE 802.11ax, 802.11ac, 802.11n, 802.11g, 802.11b, 802.11), nach einem Bluetooth-Standard (Bluetooth 1.0, 1.0B, 1.1, 1.2, 2.0, 2.0+EDR, 2.1, 2.1+EDR, 3.0, 3..0 + HS, 3.0 + DER, 4.0, 4.1, 4.2, 5.0, 5.1) oder nach einem Mobilfunk-Standard (GSM, EDGE, 3G, 4G, 5G, 6G). Insbesondere bevorzugt wird eine leistungssparende Bluetooth-Kommunikationseinrichtung, mittels derer die Auswertungseinheit mit einem Mobiltelefon oder einem anderem mobilen Endgerät gekoppelt werden kann, welches seinerseits die Auswertung der übermittelten Daten und/oder die Weiterleitung der Daten über das Internet gestattet.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt einen erfindungsgemäßen Inhalator, bestehend aus einem gattungstypischen Inhalator und einer erfindungsgemäßen Auswertungseinheit, die noch nicht an den gattungstypischen Inhalator angekoppelt ist.
Fig. 2 zeigt den erfindungsgemäßen Inhalator mit angekoppelter Auswertungseinheit.
Fig. 3 zeigt den Inhalator in geschnittener Darstellung.
Fig. 4 zeigt eine Explosionsdarstellung, aus der die Einzelkomponenten der Auswertungseinheit hervorgehen.
Fig. 5 zeigt in geschnittener Darstellung eine Durchflusssensoreinheit der Auswertungseinheit.
Fig. 6 und 7 zeigen den Inhalator bei der Nutzung im noch nicht niedergedrückten und im niedergedrückten Zustand des Containers und der Auswertungseinheit.
Fig. 8 und 9 zeigen mit verschiedenen Schnitten die Strömungspfade der Luft während der Nutzung des Inhalators.

### DETAILLIERTE BESCHREIBUNG DER AUSFÜHRUNGSBEISPIELE

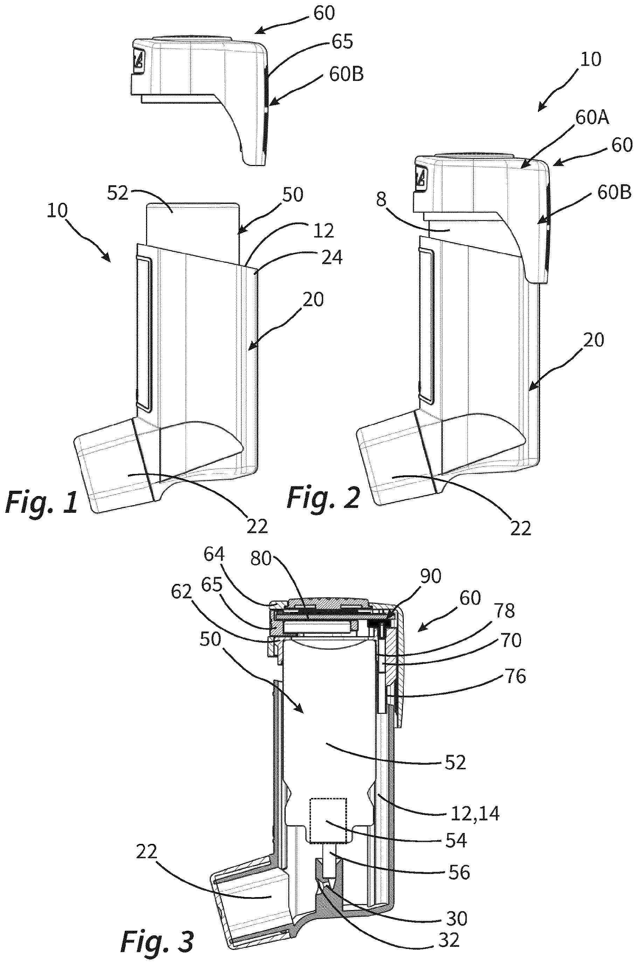

Fig. 1 zeigt einen erfindungsgemäßen Inhalator 10, der sich aus einem im Wesentlichen gattungstypischen Inhalator sowie einer erfindungsgemäß ausgestalteten Auswertungseinheit 60 zusammensetzt.

Fig. 2 zeigt den Inhalator 10 nach Ergänzung um die Auswertungseinheit 60. Figur 3 zeigt eine geschnittene Darstellung hiervon.

Der im Wesentlichen gattungstypisch geartete Inhalator umfasst ein Gehäuse 20, welches in der für MDI-Spender üblichen Art und Weise etwa eine L-Form aufweist. Das Gehäuse 20 verfügt insbesondere über einen vertikalen Hauptabschnitt, der einen Aufnahmeraum 12 für eine Containereinheit 50 bildet, sowie über ein gegenüber dem Hauptabschnitt angewinkeltes Mundstück 22, welches in Fig. 1 mit aufgesetzter Schutzkappe dargestellt ist.

Der Aufnahmeraum 12 wird durch eine Aufnahmeraumwandung 24 begrenzt und ist nach oben offen ausgestaltet, so dass hier eine Containereinheit 50 eingeschoben werden kann. Bezug nehmend auf Fig. 3 ist zu ersehen, dass die Containereinheit 50 über einen zur Aufnahme von Flüssigkeit vorgesehenen Container 52 sowie einen Auslassstutzen 56 verfügt. Der Auslassstutzen 56 kann gegen die Kraft einer Ventilfeder eines Auslassventils 54 in Richtung des Containers 52 verlagert werden und öffnet dabei das Auslassventil 54. Wie in Fig. 3 zu ersehen ist, ist die Containereinheit 50 in einer Überkopflage in dem Aufnahmeraum 12 aufgenommen. Der Auslassstutzen 56 ragt in eine Aufnahmestruktur des Gehäuses 20, die einen Austragkanal 30 sowie eine Austragöffnung 32 am Ende des Austragkanals 30 aufweist. Wird der Container 52 niedergedrückt, so wird Flüssigkeit durch den Auslassstutzen 56 und den Austragkanal 30 sowie die Austragöffnung 32 in Richtung des Mundstücks 22 abgegeben und vermengt sich mit Luft, die vom Benutzer zeitgleich eingeatmet wird. Diese Luft wird durch einen Ringspalt 14 zwischen dem Container 52 und der Aufnahmeraumwandung 24 in den Inhalator 10 eingesogen.

Die in Fig. 1 noch nicht aufgesetzte Auswertungseinheit 60 verfügt über einen Kopfabschnitt 60A, der sich im aufgesetzten Zustand der Fig. 2 und 3 zum überwiegenden Teil oberhalb des Containers 52 befindet, sowie über einen seitlich hiervon hinabragenden Schürzenabschnitt 60B. In einer den nachfolgenden Figuren noch zu entnehmenden Weise ist der Schürzenabschnitt 60B korrespondierend zur Aufnahmeraumwandung 24 beschaffen, so dass er in dem aufgesetzten Zustand der Fig. 2 und 3 verhindert, dass die Auswertungseinheit 60 als Ganzes um die Hauptachse des Containers 52 verdrehbar ist. Auch bei aufgesetzter Auswertungseinheit 60 verbleibt ein breiter Einströmspalt 8 zwischen der Auswertungseinheit 60 und einem oberen Rand der Aufnahmeraumwandung 24. Dieser Abstand ist von Relevanz, da er dafür Sorge trägt, dass ein Inhalieren von Luft durch den Benutzer durch den Ringspalt 14 gegen einen nur geringen Strömungswiderstand zu erfolgen hat.

Wie der Fig. 3 zu entnehmen ist und in den folgenden Darstellungen noch näher erläutert wird, verfügt die Auswertungseinheit 60 über einen Messkanalstutzen 76, der von einem Messkanal 70 durchdrungen ist und bis in den Ringspalt 14 zwischen der Aufnahmeraumwandung 24 und dem Container 52 ragt. Weiterhin verfügt die Auswertungseinheit 60 über einen Einschubbereich 78, der so an die Dimensionen des Containers 52 angepasst ist, dass er mit diesem eine ohne großen Kraftaufwand lösbare Klemmverbindung eingeht.

Die Fig. 3 zeigt auch, dass die Auswertungseinheit 60 nach außen primär durch ein Innenbauteil 62 und ein Außenbauteil 64 abgeschlossen ist, die gemeinsam einen Innenbereich mit einer Hauptplatine 80 sowie einer daran angebrachten Durchflusssensoreinheit 90 begrenzen.

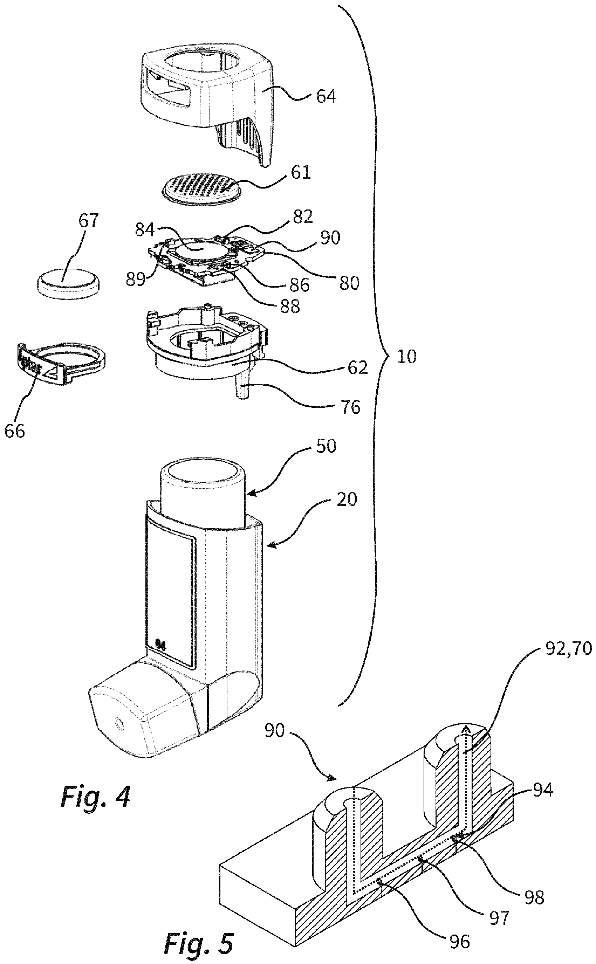

Die Einzelkomponenten der Auswertungseinheit 60 sind insbesondere der Fig. 4 zu entnehmen.

In der Fig. 4 ist zu erkennen, dass neben dem Innenbauteil 62 und dem Außenbauteil 64 die Auswertungseinheit 60 insbesondere eine Hauptplatine 80 umfasst, die in nicht näher dargestellter Weise mit dem Außenbauteil 64 verbunden wird und an ihrer Unterseite einen Aufnahmeschacht für eine Batterie 67 und deren Batterieträger 66 aufweist. Wenn der Batterieträger 66 hier eingeschoben ist, sind das Innenbauteil 62 und das Außenbauteil 64 miteinander verriegelt. Zwischen der Platine 80 und dem Außenbauteil 64 ist weiterhin eine Betätigungsfläche 61 vorgesehen, der zusammenwirkt mit einer Folie 84 zur Krafterfassung. Hierbei kann es sich insbesondere um eine Folie handeln, deren elektrischer Widerstand durch Zusammendrücken eine Veränderung erfährt. Ebenfalls auf der Platine angebracht sind ein Prozessor 82, ein Beschleunigungssensor 86 sowie ein miniaturisierter Lautsprecher 88 und eine Bluetooth-Kommunikationseinrichtung 89.

Im Kontext der Erfindung ist der wesentlichste Bestandteil auf der Platine 80 jedoch eine Durchflusssensoreinheit 90, die auf der Unterseite der Platine 80 angebracht ist und in einer demgegenüber um 180° gekippten Stellung geschnitten in Fig. 5 dargestellt ist. Diese Durchflusssensoreinheit 90 weist einen zweimal um jeweils 90° angewinkelten Sensorkanal 92 auf, der Teil des bereits genannten Messkanals 70 ist. In diesem Sensorkanal 92 ist ein Durchflusssensor 94 vorgesehen, der aus einer Mehrzahl von Elementen zusammengesetzt ist, nämlich aus zwei Temperatursensoren 96, 98, zwischen denen ein Heizelement 97 angeordnet ist. Wenn keine Luft durch den Sensorkanal 92 strömt, so bewirkt eine Erwärmung mittels des Heizelements 97 symmetrisch eine identische Erwärmung der Luft an beiden Temperatursensoren. Wenn Luft entlang der gepunkteten Linie durch die Durchflusssensoreinheit 90 strömt, so erfolgt die Erwärmung asymmetrisch. Je größer der Luftstrom ist, desto geringer ist die Temperatur am Temperatursensor 96 und desto höher ist die Temperatur am Temperatursensor 98.

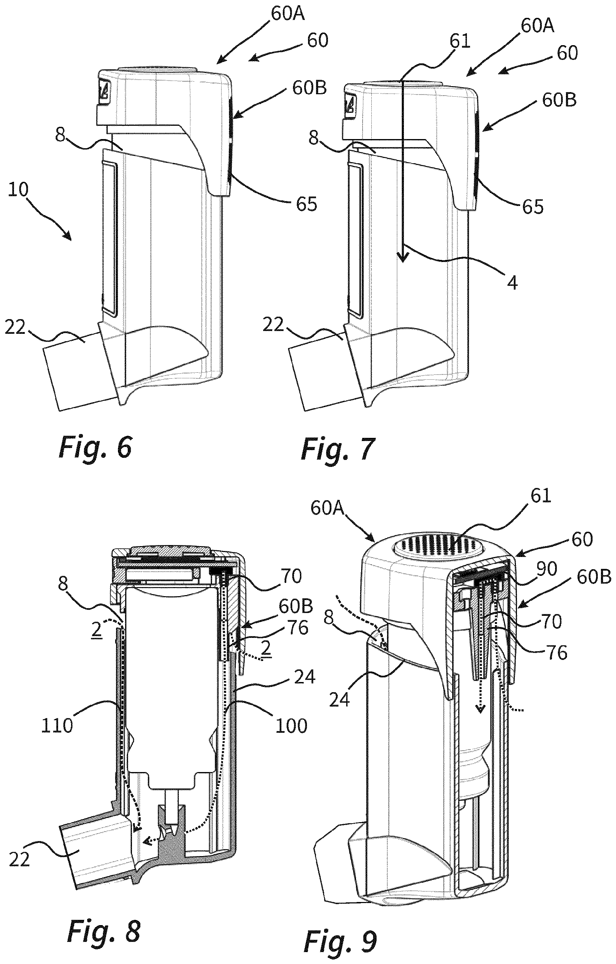

Die Fig. 6 bis 9 verdeutlichen die Verwendung des Inhalators und die dabei stattfindende Auswertung. Ausgehend vom Zustand der Fig. 6 hält der Benutzer den Inhalator 10 in der in den Figuren verdeutlichten Ausrichtung vor sein Gesicht und umschließt das Mundstück 22 mit den Lippen. Er inhaliert dann Luft und drückt in der in Fig. 7 verdeutlichten Weise mit einem Finger auf die Betätigungsfläche 61, wobei die Auswertungseinheit 60 als Ganzes gemeinsam mit dem Container 52 in Richtung des Pfeils 4 niedergedrückt wird und somit ein Austrag von zerstäubter Flüssigkeit durch die Austragöffnung 32 stattfindet.

Die derweil eingesogene Luft strömt zum weit überwiegenden Teil durch den Spalt 8 in den Ringspalt 14 zwischen dem Container 52 und der Aufnahmeraumwandung 24. Ein kleinerer Anteil der Luft strömt darüber hinaus durch die Durchbrechungen 65 im Außenbauteil 64 in den Ringspalt 14.

Bezug nehmend auf Fig. 8 ist zu erkennen, dass ein Großteil der Luft entlang des mit gestrichelter Linie dargestellten Strömungspfads 110 einströmt. Da sowohl der Einströmspalt 8 als auch der Ringspalt 14 wenig Strömungswiderstand verursachen, strömt die auf diesem Strömungspfad 110 einströmende Luft weitgehend ungehindert zum Mundstück 22. Gleichzeitig strömt jedoch auch Luft entlang eines Strömungspfads 100 zum Mundstück 22. Dieser Strömungspfad 100 verläuft durch die Durchbrechungen 65 und eine Durchbrechung des Innenbauteils 62 in den Messkanal 70. Wesentlichster Teil dieses Messkanals 70 ist der Sensorkanal 92 in der in Fig. 5 dargestellten Durchflusssensoreinheit 90. Dort wird anhand des Durchflusssensors 94 der Luftstrom gemessen. Die Luft strömt weiter in jenen Teil des Messkanals 70, der durch den bereits beschriebenen Messkanalstutzen 76 verläuft, und gelangt somit in den Ringspalt 14.

Obwohl hier ein vergleichsweise geringer Anteil des Gesamtluftstroms beider Strömungspfade 110, 100 von der entlang des Strömungspfads 100 und durch den Messkanal 70 strömenden Luft gebildet ist, vorliegend etwa 1 % bis 2 % des Gesamtluftstroms, kann aufgrund eines definierten Zusammenhangs zwischen den Luftströmen entlang der Strömungspfade 100, 110 auf den Gesamtluftstrom rückgeschlossen werden.

Dies ermöglicht es beispielsweise, dass nach Abschluss eines Inhalationsvorgangs, der mittels des Kraftsensors 84 erfasst worden ist, der Prozessor 82 der Auswertungseinheit 60 errechnen kann, ob die Inhalation in der gewünschten Weise erfolgt ist, insbesondere also, ob der Benutzer ausreichend tief eingeatmet hat und ob das Einatmen und das Auslösen des Austrags zeitlich richtig aufeinander abgestimmt waren. Ist dies der Fall, so kann mittels des Lautsprechers 88 ein entsprechendes Geräusch abgegeben werden. Ist der Austragvorgang nicht innerhalb der gewünschten Parameter erfolgt, so kann ein anderweitiges Geräusch dies für den Nutzer erkennbar verdeutlichen.

In nicht näher dargestellter Art und Weise kann neben der unmittelbaren Benachrichtigung an Benutzer auch eine Datenspeicherung oder Datenweitergabe, beispielsweise an ein Mobiltelefon, erfolgen, so dass die Daten zu einem späteren Zeitpunkt von anderen beteiligten Personen, beispielsweise dem verschreibenden Arzt, ausgewertet werden können. Hierfür kann die Kommunikationseinrichtung 89 Verwendung finden. Diese schafft insbesondere eine Verbindung zu einem Smartphone, von dem aus die Daten weitergeleitet werden können. Durch den Inhalator selbst oderdurch das Smartphone können die erfassten Daten auch um Geolokalisierungsdaten ergänzt werden.

Durch die unterschiedlichen Strömungspfade 100, 110 wird erreicht, dass gleichzeitig eine präzise Messung des inhalierten Luftstroms möglich ist, ohne dass dies zur Folge hat, dass das Inhalieren für den Benutzer deutlich erschwert ist.

## Patentansprüche

1. Inhalator (10) zur Abgabe eines inhalationsfähigen Mediums mit den folgenden Merkmalen:
a. der Inhalator (10) verfügt über ein Gehäuse (20) mit einem Mundstück (22) und einem von einer Aufnahmeraumwandung (24) umgebenen Aufnahmeraum (12) für eine Containereinheit (50), der an einem Ende offen ausgebildet ist, und
b. der Inhalator (10) verfügt über eine im Aufnahmeraum (12) angeordnete Containereinheit (50), und
c. die Containereinheit (50) verfügt über einen Auslassstutzen (56), der kommunizierend an einem Austragkanal (30) des Gehäuses (20) angeschlossen ist, sowie über einen Container (52) zur Lagerung des Mediums vor dem Austrag, wobei durch Verlagerung des Containers (52) gegenüber dem Auslassstutzen (56) ein Auslassventil (54) der Containereinheit (50) geöffnet werden kann, und
d. der Container (52) der Containereinheit (50) weist zum offenen Ende des Aufnahmeraums (12), so dass er niederdrückbar ist, um hierdurch das Auslassventil (54) zu öffnen und Medium durch den Austragkanal (30) bis zu einer Austragöffnung (32) am Ende des Austragskanal (30) austreten zu lassen, und
e. zwischen dem Container (52) und der Aufnahmeraumwandung (24) des Aufnahmeraums (12) ist ein Ringspalt (14) vorgesehen, durch den hindurch Luft während der Inhalation angesogen wird, die sich mit dem durch die Austragöffnung (32) ausströmenden Medium vermengt, und
f. der Inhalator (10) verfügt über eine Auswertungseinheit (60), die auf das vom Auslassstutzen weg weisende Ende des Containers (52) aufgesetzt ist und gemeinsam mit dem Container (52) niederdrückbar ist, wobei die Auswertungseinheit (60) zur Erfassung der am Mundstück (22) eingesogenen Luft ausgebildet ist,
**gekennzeichnet durch** die folgenden weiteren Merkmale:
g. die Auswertungseinheit (60) umfasst einen Messkanal (70) mit einem Messkanaleingang und einem Messkanalausgang, wobei der Messkanal (70) einen Teil eines ersten Strömungspfades (100) für aus einer Umgebung in das offene Ende des Aufnahmeraums einströmende Luft bildet und zwischen dem Messkanaleingang und dem Messkanalausgang über einen Durchflusssensor (94) zur Erfassung der durch den Messkanal (70) strömenden Luft verfügt, und
h. es ist ein zweiter Strömungspfad (110) für aus einer Umgebung in das offene Ende des Aufnahmeraums einströmende Luft vorgesehen, der nicht durch den Messkanal (70) führt, und
i. der erste Strömungspfad (100) und der zweite Strömungspfad (110) sind derart aufeinander abgestimmt, dass der Strömungswiderstand des ersten Strömungspfades (100) mindestens dem Strömungswiderstand des zweiten Strömungspfades (110) entspricht.

2. Inhalator (10) nach Anspruch 1 mit mindestens einem derfolgenden weiteren Merkmale:
a. der Strömungswiderstand des ersten Strömungspfades (100) von einer Umgebung (2) bis zum Mundstück (22) beträgt mindestens 1.000.000 N · s / m⁵, vorzugsweise mindestens 5.000.000 N · s / m⁵, und/oder
b. der Strömungswiderstand des zweiten Strömungspfades (110) von einer Umgebung (2) bis zum Mundstück (22) beträgt maximal 1.000.000 N · s / m⁵, vorzugsweise maximal 500.000 N · s / m⁵, und/oder
c. der Strömungswiderstand des ersten Strömungspfades (100) ist mindestens um Faktor 10 größer als der Strömungswiderstand des zweiten Strömungspfades (110), vorzugsweise mindestens um Faktor 25, insbesondere vorzugsweise mindestens um Faktor 35.

3. Inhalator (10) nach Anspruch 1 oder 2 mit dem folgenden weiteren Merkmal:
a. der Durchflusssensor (94) ist als thermischer Durchflusssensor (94) ausgebildet und verfügt über zwei Temperatursensoren (96, 98) und ein dazwischen angeordnetes Heizelement (97).

4. Inhalator (10) nach einem der vorstehenden Ansprüche mit dem folgenden weiteren Merkmal:
a. ein vom Messkanal (70) durchdrungener Messkanalstutzen (76) weist eine sich in Richtung einer Auslassöffnung des Messkanals (70) verjüngende Formgebung auf, insbesondere eine sich stetig verjüngende Formgebung.

5. Inhalator (10) nach einem der vorstehenden Ansprüche mit dem folgenden weiteren Merkmal:
a. die Auswertungseinheit (60) weist einen Kopfabschnitt (60A) sowie einen sich hiervon in einem Winkelbereich <360° in Richtung der Aufnahmeraumwandung (24) erstreckenden Schürzenabschnitt (60B) auf, wobei der Schürzenabschnitt (60B) außenseitig eines vom Messkanal (70) durchdrungenen Messkanalstutzens (76) angeordnet ist.

6. Inhalator (10) nach einem der vorstehenden Ansprüche mit dem folgenden weiteren Merkmal:
a. die Auswertungseinheit (60) und die Aufnahmeraumwandung (24) des Aufnahmeraums (12) sind bezüglich ihrer Formgebung derart aufeinander abgestimmt, dass die Auswertungseinheit (60) in einer definierten Drehstellung oder einem definierten Drehstellungsbereich auf den Container (52) aufsetzbar ist,
insbesondere mit mindestens einem der folgenden zusätzlichen Merkmale:
b. die Auswertungseinheit (60) weist einen Kopfabschnitt (60A) sowie einen sich hiervon in Richtung der Aufnahmeraumwandung (24) erstreckenden Schürzenabschnitt (60B) auf, wobei der Schürzenabschnitt (60B) eine nach innen weisende Formgebung aufweist, die an eine nicht rotationssymmetrische Außenform der Aufnahmeraumwandung (24) drehsichernd formschlüssig angepasst ist, und/oder
c. der Ringspalt (14) zwischen dem Container (52) und einer Innenseite der Aufnahmeraumwandung (24) weist eine nicht einheitliche lichte Weite auf und ist nicht umlaufend ausreichend für die Aufnahme eines vom Messkanal (70) durchdrungenen Messkanalstutzens (76) dimensioniert, und/oder
d. in der Drehstellung oder dem Drehstellungsbereich, in dem die Auswertungseinheit (60) auf den Container (52) aufsetzbar ist, ist der Messkanal (70) gegenüberliegend zum Mundstück (22) angeordnet.

7. Inhalator (10) nach einem der vorstehenden Ansprüche mit dem folgenden weiteren Merkmal:
a. ein Messkanalausgang des Messkanals (70) ist zumindest bei niedergedrücktem Container (52) im Ringspalt (14) angeordnet.

8. Inhalator (10) nach einem der vorstehenden Ansprüche mit dem folgenden weiteren Merkmal:
a. die Auswertungseinheit (60) weist eine Durchflusssensoreinheit (90) auf, die über ein Sensorgehäuse mit nichtlinearem Sensorkanal (92) verfügt, insbesondere mit einem zweimal um jeweils 90° angeknickten Sensorkanal (92).

9. Inhalator (10) nach einem der vorstehenden Ansprüche mit dem folgenden weiteren Merkmal:
a. ein Gehäuse (62, 64) der Auswertungseinheit (60) weist mindestens eine Durchbrechung (65) zum Einströmen der Luft in den Messkanal (70) auf.

10. Inhalator (10) nach einem der vorstehenden Ansprüche mit dem folgenden weiteren Merkmal:
a. der zweite Strömungspfad (110) verläuft durch einen Einströmspalt (8), der auf der einen Seite durch ein oberes Ende der Aufnahmeraumwandung (24) und auf der anderen Seite durch einen Kopfabschnitt (60A) der Auswertungseinheit (60) gebildet wird.

11. Inhalator (10) nach einem der vorstehenden Ansprüche mit dem folgenden weiteren Merkmal:
a. die Auswertungseinheit (60) weist eine Steuerschaltung auf, die Ausgangssignale des Durchflusssensors (94) erhält und zur Auswertung dieser Strömungssignale vorgesehen ist.

12. Inhalator (10) nach einem der vorstehenden Ansprüche mit mindestens einem der folgenden weiteren Merkmale:
a. die Auswertungseinheit (60) verfügt über ein einstückiges Innenbauteil (62), welches einen Einschubbereich (78) zur Befestigung am Container (52) unmittelbar begrenzt und welches zumindest abschnittsweise den Messkanal (70) definiert, insbesondere vom Messkanal (70) durchdrungen ist, und/oder
b. die Auswertungseinheit weist einen mindestens zweiteiligen Befestigungsabschnitt auf, der einen Einschubbereich begrenzt, wobei durch Verlagerung der zwei Teile die Größe des Einschubbereichs anpassbar ist, und/oder
c. die Auswertungseinheit (60) weist eine Aufnahme für eine Batterie (67), insbesondere eine Knopfzelle, auf, wobei vorzugsweise ein ausziehbarer Batterieträger (66) zur Aufnahme der Batterie vorgesehen ist und wobei insbesondere vorzugsweise der Batterieträger (66) einen Riegelabschnitt bildet, mit dem ein Innenbauteil (62) und ein Außenbauteil (64) der Auswertungseinheit (60) miteinander verriegelbar sind.

13. Inhalator (10) nach einem der vorstehenden Ansprüche mit mindestens einem der folgenden weiteren Merkmale:
a. die Auswertungseinheit (60) verfügt über einen Sensor (86) zur Erfassung von Beschleunigungen und/oder zur Erfassung einer Schüttelbewegung, und/oder
b. die Auswertungseinheit (60) verfügt über einen Sensor (84) zur Erfassung einer von oben auf die Auswertungseinheit ausgeübten Betätigungskraft relativ zum Container (52), insbesondere in Form einer Sensorfolie, die bei Kompression ihren elektrischen Widerstand ändert, und/oder
c. die Auswertungseinheit (60) verfügt über eine Ausgabeeinrichtung (88) in Art eines Displays, in Art mindestens eines Signallichts und/oder eines akustischen Signalgebers, und/oder
d. die Auswertungseinheit (60) verfügt über eine Kommunikationseinrichtung (89) zur Kommunikation mit einer externen Kommunikationsstelle, insbesondere in Art eines mobilen Endgeräts wie eines Mobiltelefons, wobei die Kommunikationseinrichtung (89) vorzugweise zur Kommunikation nach einem WIFI-Standard, einem Bluetooth-Standard oder einem Mobilfunkstandard ausgebildet ist.

## Claims

1. Inhaler (10) for dispensing an inhalable medium, with the following features:
a. the inhaler (10) has a housing (20) with a mouthpiece (22) and with a chamber (12) for a container unit (50), which chamber (12) is surrounded by a chamber wall (24) and is open at one end, and
b. the inhaler (10) has a container unit (50) arranged in the chamber (12), and
c. the container unit (50) has an outlet connector (56) which is attached to a discharge channel (30) of the housing (20) in a communicating manner, and also a container (52) for storing the medium before discharge, wherein, by moving the container (52) with respect to the outlet connector (56), an outlet valve (54) of the container unit (50) can be opened, and
d. the container (52) of the container unit (50) faces the open end of the chamber (12) so that it can be pressed down in order thereby to open the outlet valve (54) and allow medium to pass through the discharge channel (30) to a discharge opening (32) at the end of the discharge channel (30), and
e. an annular gap (14) is provided between the container (52) and the chamber wall (24) of the chamber (12), through which annular gap (14) air is sucked in during inhalation and mixes with the medium flowing out through the discharge opening (32), and
f. the inhaler (10) has an evaluation unit (60) which is placed onto that end of the container (52) facing away from the outlet connector and can be pressed down together with the container (52), wherein the evaluation unit (60) is designed to detect the air sucked in at the mouthpiece (22),
**characterized by** the following further features:
g. the evaluation unit (60) comprises a measuring channel (70) with a measuring channel inlet and a measuring channel outlet, wherein the measuring channel (70) forms part of a first flow path (100) for air flowing into the open end of the chamber from an environment and, between the measuring channel inlet and the measuring channel outlet, has a flow sensor (94) for detecting the air flowing through the measuring channel (70), and
h. a second flow path (110) is provided for air flowing into the open end of the chamber from an environment, which second flow path (110) does not lead through the measuring channel (70), and
i. the first flow path (100) and the second flow path (110) are matched to each other in such a way that the flow resistance of the first flow path (100) corresponds at least to the flow resistance of the second flow path (110).

2. Inhaler (10) according to Claim 1, with at least one of the following further features:
a. the flow resistance of the first flow path (100) from an environment (2) to the mouthpiece (22) measures at least 1,000,000 N·s/m⁵, preferably at least 5,000,000 N·s/m⁵, and/or
b. the flow resistance of the second flow path (110) from an environment (2) to the mouthpiece (22) measures a maximum of 1,000,000 N·s/m⁵, preferably a maximum of 500,000 N·s/m⁵, and/or
c. the flow resistance of the first flow path (100) is at least a factor of 10 greater than the flow resistance of the second flow path (110), preferably at least a factor of 25, particularly preferably at least a factor of 35.

3. Inhaler (10) according to Claim 1 or 2, with the following further feature:
a. the flow sensor (94) is designed as a thermal flow sensor (94) and has two temperature sensors (96, 98) and, arranged between them, a heating element (97).

4. Inhaler (10) according to one of the preceding claims, with the following further feature:
a. a measuring channel connector (76) through which the measuring channel (70) extends has a shape that tapers in the direction of an outlet opening of the measuring channel (70), in particular a continuously tapering shape.

5. Inhaler (10) according to one of the preceding claims, with the following further feature:
a. the evaluation unit (60) has a head portion (60A) and an apron portion (60B) extending therefrom in an angular range < 360° in the direction of the chamber wall (24), wherein the apron portion (60B) is arranged outside a measuring channel connector (76) through which the measuring channel (70) extends.

6. Inhaler (10) according to one of the preceding claims, with the following further feature:
a. the evaluation unit (60) and the chamber wall (24) of the chamber (12) are matched to each other in terms of their shape, in such a way that the evaluation unit (60) can be placed onto the container (52) in a defined rotational position or a defined rotational position range,
in particular with at least one of the following additional features:
b. the evaluation unit (60) has a head portion (60A), and an apron portion (60B) extending therefrom in the direction of the chamber wall (24), wherein the apron portion (60B) has an inwardly facing shape that is adapted for form-fit engagement on a non-rotationally symmetrical outer shape of the chamber wall (24) to provide locking against rotation, and/or
c. the annular gap (14) between the container (52) and an inner face of the chamber wall (24) has a non-uniform inside width and is not sufficiently dimensioned circumferentially to accommodate a measuring channel connector (76) through which the measuring channel (70) extends, and/or
d. in the rotational position or the rotational position range in which the evaluation unit (60) can be placed onto the container (52), the measuring channel (70) is arranged opposite the mouthpiece (22).

7. Inhaler (10) according to one of the preceding claims, with the following further feature:
a. a measuring channel outlet of the measuring channel (70) is arranged in the annular gap (14) at least when the container (52) is pressed down.

8. Inhaler (10) according to one of the preceding claims, with the following further feature:
a. the evaluation unit (60) has a flow sensor unit (90) which has a sensor housing with a non-linear sensor channel (92), in particular with a sensor channel (92) that is bent twice, in each case by 90°.

9. Inhaler (10) according to one of the preceding claims, with the following further feature:
a. a housing (62, 64) of the evaluation unit (60) has at least one aperture (65) for air to flow into the measuring channel (70).

10. Inhaler (10) according to one of the preceding claims, with the following further feature:
a. the second flow path (110) runs through an inflow gap (8), which is formed on the one hand by an upper end of the chamber wall (24) and on the other hand by a head portion (60A) of the evaluation unit (60).

11. Inhaler (10) according to one of the preceding claims, with the following further feature:
a. the evaluation unit (60) has a control circuit which receives output signals from the flow sensor (94) and is provided to evaluate these flow signals.

12. Inhaler (10) according to one of the preceding claims, with at least one of the following further features:
a. the evaluation unit (60) has a one-piece inner component (62) which directly delimits an insertion region (78) for fastening to the container (52) and which at least in part defines the measuring channel (70), in particular has the measuring channel (70) extending through it, and/or
b. the evaluation unit has an at least two-part fastening portion which delimits an insertion region, the size of the insertion region being adaptable by moving the two parts, and/or
c. the evaluation unit (60) has a receptacle for a battery (67), in particular a button cell, wherein a pull-out battery carrier (66) is preferably provided to accommodate the battery, and wherein the battery carrier (66) particularly preferably forms a locking portion with which an inner component (62) and an outer component (64) of the evaluation unit (60) can be locked on to each other.

13. Inhaler (10) according to one of the preceding claims, with at least one of the following further features:
a. the evaluation unit (60) has a sensor (86) for detecting accelerations and/or for detecting a shaking movement, and/or
b. the evaluation unit (60) has a sensor (84) for detecting an actuation force, exerted on the evaluation unit from above, relative to the container (52), in particular in the form of a sensor film that changes its electrical resistance when compressed, and/or
c. the evaluation unit (60) has an output device (88) in the form of a display, in the form of at least one signal light and/or an acoustic signal generator, and/or
d. the evaluation unit (60) has a communication device (89) for communication with an external communication site, in particular in the form of a mobile terminal such as a cell phone, wherein the communication device (89) is preferably designed for communication according to a Wi-Fi standard, a Bluetooth standard or a mobile radio standard.

## Revendications

1. Inhalateur (10) pour la distribution d'un milieu inhalable, présentant les caractéristiques suivantes :
a. l'inhalateur (10) présente un boîtier (20) avec un embout buccal (22) et un espace de réception (12) entouré d'une paroi (24) d'espace de réception pour une unité (50) formant récipient, qui est conçue pour être ouverte à une extrémité, et
b. l'inhalateur (10) présente une unité (50) formant récipient disposée dans l'espace de réception (12), et
c. l'unité (50) formant récipient a une tubulure de sortie (56) qui est raccordée de manière communicante à un canal de distribution (30) du boîtier (20), ainsi qu'un conteneur (52) pour le stockage du milieu avant la distribution, une soupape de sortie (54) de l'unité (50) formant récipient étant apte à être ouverte par le déplacement du conteneur (52) en face de la tubulure de sortie (56), et
d. le conteneur (52) de l'unité (50) formant récipient fait face à l'extrémité ouverte de l'espace de réception (12) de sorte qu'il peut être enfoncé afin d'ouvrir ainsi la soupape de sortie (54) et permettre au milieu de s'échapper par le canal de décharge (30) jusqu'à une ouverture de décharge (32) à l'extrémité du canal de décharge (30), et
e. un espace annulaire (14) est prévu entre le conteneur (52) et la paroi (24) de l'espace de réception (12), à travers lequel de l'air est aspiré pendant l'inhalation, lequel se mélange avec le milieu s'écoulant à travers l'ouverture de décharge (32), et
f. l'inhalateur (10) présente une unité d'évaluation (60) qui est placée à l'extrémité du conteneur (52) opposée à la tubulure de sortie et qui peut être enfoncée en même temps que le conteneur (52), l'unité d'évaluation (60) étant conçue pour détecter l'air aspiré au niveau de l'embout buccal (22), **caractérisé par** les autres caractéristiques suivantes :
g. l'unité d'évaluation (60) comprend un canal de mesure (70) avec une entrée de canal de mesure et une sortie de canal de mesure, le canal de mesure (70) formant une partie d'un premier chemin d'écoulement (100) pour l'air s'écoulant d'un environnement vers l'extrémité ouverte de l'espace de réception et présentant entre l'entrée de canal de mesure et la sortie de canal de mesure un capteur de débit (94) pour la détection de l'air s'écoulant à travers le canal de mesure (70), et
h. il est prévu un deuxième chemin d'écoulement (110) pour l'air s'écoulant, à partir d'un environnement, dans l'extrémité ouverte de l'espace de réception, qui ne passe pas par le canal de mesure (70), et
i. le premier chemin d'écoulement (100) et le deuxième chemin d'écoulement (110) sont adaptés l'un à l'autre de telle sorte que la résistance à l'écoulement du premier chemin d'écoulement (100) correspond au moins à la résistance à l'écoulement du deuxième chemin d'écoulement (110).

2. Inhalateur (10) selon la revendication 1, ayant au moins une des caractéristiques supplémentaires suivantes :
a. la résistance à l'écoulement du premier trajet d'écoulement (100) depuis un environnement (2) jusqu'à l'embout buccal (22) est d'au moins 1.000.000 N·s/m⁵, de préférence d'au moins 5.000.000 N·s/m⁵, et/ou
b. la résistance à l'écoulement du deuxième chemin d'écoulement (110) depuis un environnement (2) jusqu'à l'embout buccal (22) est au maximum de 1.000.000 N·s/m⁵, de préférence au maximum de 500.000 N·s/m⁵, et/ou
c. la résistance à l'écoulement du premier chemin d'écoulement (100) est supérieure d'au moins un facteur 10 à la résistance à l'écoulement du deuxième chemin d'écoulement (110), de préférence d'au moins un facteur 25, de manière particulièrement préférée d'au moins un facteur 35.

3. Inhalateur (10) selon la revendication 1 ou 2, ayant la caractéristique supplémentaire suivante :
a. le capteur de débit (94) est réalisé sous la forme d'un capteur de débit thermique (94) et comporte deux capteurs de température (96, 98) et un élément chauffant (97) disposé entre eux.

4. Inhalateur (10) selon l'une des revendications précédentes, ayant la caractéristique supplémentaire suivante :
a. une tubulure (76) de canal de mesure traversée par le canal de mesure (70) présente une forme qui se rétrécit en direction d'une ouverture de sortie du canal de mesure (70), en particulier une forme qui se rétrécit de manière continue.

5. Inhalateur (10) selon l'une des revendications précédentes, ayant la caractéristique supplémentaire suivante :
a. l'unité d'évaluation (60) présente une partie tête (60A) ainsi qu'une partie jupe (60B) s'étendant à partir de celle-ci sur une plage angulaire <360° en direction de la paroi (24) de l'espace de réception, la partie jupe (60B) étant disposée sur le côté extérieur d'une tubulure (76) de canal de mesure traversée par le canal de mesure (70).

6. Inhalateur (10) selon l'une des revendications précédentes, ayant la caractéristique supplémentaire suivante :
a. l'unité d'évaluation (60) et la paroi (24) de l'espace de réception (12) sont adaptées l'une à l'autre en termes de forme de telle sorte que l'unité d'évaluation (60) peut être placée sur le conteneur (52) dans une position de pivotement définie ou une plage de positions de pivotement définie, notamment avec au moins l'une des caractéristiques supplémentaires suivantes :
b. l'unité d'évaluation (60) présente une partie tête (60A) ainsi qu'une partie jupe (60B) s'étendant depuis celle-ci en direction de la paroi (24) de l'espace de réception, la partie jupe (60B) présentant une forme orientée vers l'intérieur qui est adaptée par complémentarité de forme à une forme extérieure non symétrique en rotation de la paroi (24) de l'espace de réception, et/ou
c. l'espace annulaire (14) entre le conteneur (52) et une face intérieure de la paroi (24) de l'espace de réception présente une largeur intérieure non uniforme et n'est pas suffisamment dimensionné sur tout le pourtour pour recevoir une tubulure (76) de canal de mesure traversée par le canal de mesure (70), et/ou
d. dans la position de pivotement ou la plage de positions de pivotement dans laquelle l'unité d'évaluation (60) peut être placée sur le conteneur (52), le canal de mesure (70) est disposé en face de l'embout buccal (22).

7. Inhalateur (10) selon l'une des revendications précédentes, ayant la caractéristique supplémentaire suivante :
a. une sortie de canal de mesure du canal de mesure (70) est agencée dans l'espace annulaire (14) au moins lorsque le conteneur (52) est enfoncé.

8. Inhalateur (10) selon l'une des revendications précédentes, ayant la caractéristique supplémentaire suivante :
a. l'unité d'évaluation (60) présente une unité (90) formant capteur de débit qui présente un boîtier de capteur ayant un canal de capteur non linéaire (92), en particulier ayant un canal de capteur (92) coudé deux fois à 90°.

9. Inhalateur (10) selon l'une des revendications précédentes, ayant la caractéristique supplémentaire suivante :
a. un boîtier (62, 64) de l'unité d'évaluation (60) présente au moins un passage (65) pour l'écoulement de l'air dans le canal de mesure (70).

10. Inhalateur (10) selon l'une des revendications précédentes, ayant la caractéristique supplémentaire suivante :
a. le deuxième chemin d'écoulement (110) passe par une fente d'entrée (8) qui est formée d'un côté par une extrémité supérieure de la paroi de l'espace de réception (24) et de l'autre côté par une partie tête (60A) de l'unité d'évaluation (60).

11. Inhalateur (10) selon l'une des revendications précédentes, présentant la caractéristique supplémentaire suivante :
a. l'unité d'évaluation (60) présente un circuit de commande qui reçoit des signaux de sortie du capteur de débit (94) et qui est prévu pour évaluer ces signaux de débit.

12. Inhalateur (10) selon l'une des revendications précédentes, ayant au moins l'une des caractéristiques supplémentaires suivantes :
a. l'unité d'évaluation (60) présente un composant intérieur (62) d'une seule pièce, qui délimite directement une zone d'insertion (78) pour la fixation sur le conteneur (52) et qui définit au moins partiellement le canal de mesure (70), en particulier qui est traversé par le canal de mesure (70), et/ou
b. l'unité d'évaluation présente une section de fixation en au moins deux parties, qui délimite une zone d'insertion, la taille de la zone d'insertion pouvant être adaptée par déplacement des deux parties, et/ou
c. l'unité d'évaluation (60) présente un logement pour une pile (67), notamment une pile bouton, un support de pile (66) extractible étant de préférence prévu pour recevoir la pile et le support de pile (66) formant notamment de préférence une partie de verrouillage avec laquelle un composant intérieur (62) et un composant extérieur (64) de l'unité d'évaluation (60) peuvent être verrouillés l'un à l'autre.

13. Inhalateur (10) selon l'une des revendications précédentes, ayant au moins l'une des caractéristiques supplémentaires suivantes :
a. l'unité d'évaluation (60) présente un capteur (86) pour détecter des accélérations et/ou pour détecter un mouvement de secousse, et/ou
b. l'unité d'évaluation (60) présente un capteur (84) pour détecter une force d'actionnement exercée par le haut sur l'unité d'évaluation par rapport au conteneur (52), en particulier sous la forme d'un film capteur qui modifie sa résistance électrique en cas de compression, et/ou
c. l'unité d'évaluation (60) présente un dispositif de sortie (88) sous la forme d'un écran d'affichage, sous la forme d'au moins un signal lumineux et/ou d'un émetteur de signaux acoustiques, et/ou
d. l'unité d'évaluation (60) présente un dispositif de communication (89) pour communiquer avec un point de communication externe, en particulier sous la forme d'un terminal mobile tel qu'un téléphone portable, le dispositif de communication (89) étant de préférence conçu pour communiquer selon un standard WIFI, un standard Bluetooth ou un standard de téléphonie mobile.
